# EUROPEAN PATENT APPLICATION

(11) **EP 4 674 951 A1**
(43) Date of publication of application: **07.01.2026**
(21) Application number: 24763310.0
(22) Date of filing: 27.02.2024
(51) Int. Cl.: C12N 5/02

(54) **MURINE EXTRACELLULAR MATRIX AND USE THEREOF IN CELL CULTURES**

(30) Priority: 27.02.2023 ES 202330170
(71) Applicant: Universidad De Malaga, 29071 Málaga (ES)
(72) Inventor: PÉREZ POMARES, Jose María, 29590 Málaga Málaga (ES); RUIZ VILLALBA, Adrián, 29590 Málaga Málaga (ES); DA COSTA OLIVEIRA, Claudia Patricia, 29590 Málaga Málaga (ES); GUADIX DOMÍNGUEZ, Juan Antonio, 29590 Málaga Málaga (ES)
(74) Representative: San Martin Alarcia, Esther
(86) International application number: PCT/ES2024/070114
(87) International publication number: WO 2024/180274

(57) **Abstract**

The present invention relates to an extracellular matrix obtained from an EPIC murine cell line, the method of obtaining same and uses in cell cultures.

## Description

### Field of the Invention

The invention falls within the field of cell cultures, specifically referring to a murine extracellular matrix (ECM) secreted by a cell line of epicardial origin, a method for obtaining it, and its use.

### Background

The ECM is a fundamental element in cell signalling as it acts as a trigger for specific cellular responses to a wide variety of environmental signals. In fact, all cell interaction processes require a specific environment that is specific to the organ under study. In the case of the heart, the synthesis, deposition, and degradation of the ECM are necessary for the effective attachment, migration, and differentiation of a plethora of different cardiac cell types (cardiomyocytes, endothelial and smooth muscle cells, cardiac fibroblasts, neurons, pericytes, circulating cells) during heart formation. During adulthood, this complexity increases, especially in pathological conditions such as those leading to ventricular remodelling after damage.

In these contexts, the epicardium, the outermost layer of the heart, has an essential role. During embryogenesis, the embryonic epicardium is the first external cellular source of endothelial and smooth muscle cells that form the coronary system of the heart, along with cardiac fibroblasts, the interstitial cells responsible for the synthesis, secretion, and degradation of the ECM. All these epicardium-derived cells (EPDCs) migrate to the myocardium after undergoing an epithelial-mesenchymal transition (EMT) and also produce part of the native cardiac ECM as they differentiate. In adulthood, epicardium-derived cardiac fibroblasts are responsible for the ventricular remodelling process that occurs after a myocardial infarction. Ruiz-Villalba et al, JACC, 2015 - doi: 10.1016/j.jacc.2015.03.

### Native ECM reproduces the cellular microenvironment of its tissue source

Currently, biomaterial studies are being explored for the assembly of synthetic ECM, both for *in vitro* cell matrix studies and for therapeutic replacements. However, some of these biomaterials fail to develop the necessary molecular complexity and organisation found in native ECM. The use of de-cellularised ECM from whole organs has also been studied, which is of interest for studies of native ECM architecture. However, its uncontrolled variability and the availability of tissue sources are limitations that weaken its viability for future basic and clinical applications. Therefore, there are different strategies and protocols to improve the isolation of native ECM *in vitro.*

When using native ECM substrates, the cellular microenvironment is more suitable and better preserved than when using artificial matrices. However, native proteins from primary cultures (such as cardiac fibroblasts) or de-cellularised tissues require a large number of animals to obtain a sufficient quantity of molecules for characterisation and application in various studies, which goes against the principle of the "3Rs".

On the other hand, the availability of native epicardial tissue, due to its limited cell number, restricts studies on the composition of epicardium-derived ECM. Primary epicardial cultures are difficult to establish and have a short half-life. Therefore, the analysis of epicardium-derived ECM is highly dependent on the use of continuous epicardial cell lines for the isolation of significant amounts of ECM proteins.

A few continuous (immortal) epicardium-derived cell lines have been described in the state of the art (Austin et al, Dev Dyn., 2008 - DOI: 10.1002/dvdy.21421; Wada et al, Circ. Res., 2003 - doi: 10.1161/01.RES.0000060484.11032.0B). However, these cell lines have some limitations.

On the one hand, the epicardium-derived cell line described by Austin was generated from the epicardium of embryonic murine atria of mouse at E12.5. At this stage, the multipotent capacity of the epicardium is reduced (EMT begins at E11.5) (Ruiz-Villalba et al, PLoS One, 2013 - doi: 10.1371/journal.pone.0053694). Furthermore, the biological properties of the atrial epicardium are not comparable to those of the ventricular epicardium, as only the latter actively participates in the development of the nascent cardiac interstitium and it will be responsible for the response to the most common pathologies in adults, such as ischemic disease or dilated cardiomyopathy.

On the other hand, Wada immortalised a pre-existing cell line, from epicardium isolated from adult rats, by transfection. The authors did not detail the complete procedure. Furthermore, this cell line has a more epithelial profile rather than a mesenchymal nature. Both properties significantly reduce the native nature of this cell line.

The inventors of this patent generated the immortalised EPIC cell line from a primary culture of embryonic epicardium isolated from E11.5-day-old mice (Ruiz-Villalba A, Ziogas A, Ehrbar M, Pérez-Pomares JM. Characterization of epicardial-derived cardiac interstitial cells: differentiation and mobilization of heart fibroblast progenitors. PLoS One. 2013;8(1):e53694. doi:10.1371/journal.pone.0053694).

Currently, the EPIC line is the one that best reproduces the biological properties of the native epicardium and cells derived from it, without significant limitations associated with primary embryonic epicardial cell cultures, such as biological and technical variability among experiments and the limited amount of proteins recovered from the ECM.

The ECM of the present invention derived from the EPIC cell line exhibits native structural and molecular properties and improved circulating cell retention capacity compared to other commercial immortalised cell lines.

### Matrigel^{®} as the gold standard for research on the market

Basal membranes are acellular types of ECM that underlie most epithelial and endothelial cells and are composed primarily of laminins, proteoglycans, collagen IV, growth factors, cytokines, and chemokines. Their biological function is crucial in providing physical and biological signals to overlying endothelial and epithelial cells, which is why artificial basal membrane materials are widely used in *in vitro* approaches. One of the most widely used basal membrane-derived materials in both basic and clinical research is Matrigel^{®}. Matrigel^{®} is derived from the basal membrane of the Engelbreth-Holm-Swarm (EHS) murine tumour, which produces extraordinary amounts of ECM readily available under non-denaturing conditions. Since most tumours are composed of this acellular membrane, commercially available Matrigel^{®} became popular in angiogenesis and cancer research. To obtain Matrigel^{®}, EHS tumour tissue is thoroughly washed to remove cellular and serum-derived proteins, and its ECM is extracted using urea to break protein-protein interactions. The resulting matrix is finally dialysed to concentrate the ECM. Kibbey, M.C. Maintenance of the EHS sarcoma and Matrigel preparation. Journal of Tissue Culture Methods 16, 227-230 (1994). https://doi.org/10.1007/BF01540656.

The present invention discloses a comparative analysis of the protein content between Matrigel^{®} and the ECM of the present invention, which shows little overlap between the composition of both matrices, most of the proteins identified in Matrigel^{®} labelled as extracellular proteins involved in processes mainly related to the regulation of enzymatic activity.

The ECM of the present invention also presents proteins related to angiogenesis and cell proliferation processes in the set of biological processes with the greatest statistical significance, while these terms do not appear in the processes annotated to the most significant biological Gene Ontology (GO) terms obtained from the study of Matrigel^{®} proteins using proteomics. This demonstrates that the ECM of the present invention is much better for cell culture growth.

### Description

In this report, "EPIC IM" and "ECM IM" are synonymous and are used interchangeably.

The present invention relates to a murine extracellular matrix (ECM) obtained from the EPIC cell line, said cell line being deposited at the Leibniz Institute DSMZ (German Collection of Microorganisms and Cell Cultures, Department of Human and Animal Cell Lines), Inhoffenstraße 7 B, 38124 Braunschweig, Germany, with deposit number DSM ACC3373, or a murine extracellular matrix obtained from mutants derived from said cell line, wherein said mutants have the same or better properties than the DSM ACC3373 cell line.

This strain was deposited on 29 September 2022 at the Leibniz Institute DSMZ (German Collection of Microorganisms and Cell Cultures, Department of Human and Animal Cell Lines), Inhoffenstraße 7 B, 38124 Braunschweig, Germany, in accordance with the provisions of the Budapest Treaty.

Mutants derived from this cell line refer to cell lines that are derived from DSM ACC3373, or obtained from DSM ACC3373, and may have mutations compared to the EPIC line, preferably, the murine extracellular matrix obtained from the mutant line has the same or better properties than the DSM ACC3373 cell line.

Preferably, the mutant derived from DSM ACC3373 has at least 80% or more, preferably at least 90% or more, more preferably at least 95% or more, or even up to 100% or more of the properties compared to the DSM ACC3373 cell line under equal conditions.

These properties refer to the ability to induce angiogenic-type processes and/or cell proliferation in another cell line.

In a particular embodiment, the ECM of the invention can be divided into a soluble fraction (EPIC-SM) and an insoluble fraction (EPIC-IM).

The usual methods for isolating ECM from the cell line from which it originates comprise disrupting the plasma membrane of these cells. The disruption may be chemical, mechanical, or a combination of both techniques. Each method leads to different de-cellularisation efficiencies, based on contamination with intracellular components and ECM retention, depending on the cell line of origin.

Depending on the method of ECM isolation used, the soluble and insoluble fractions can be obtained separately and then combined, or both fractions can be isolated simultaneously already combined.

In one particular embodiment, ECM from the EPIC cell line is obtained by chemical disruption with NH₄OH; the NH4OH solution contains debris and other cellular remains.

In one particular embodiment, the obtaining of ECM comprises:
1) de-cellularisation of the EPIC cell line with deposit number DSM ACC3373 attached to a culture plate using an NH₄OH solution for at least 5 minutes,
2) collecting and discarding the NH₄OH solution from the previous step, such that the ECM remains attached to the plate.

The advantage of this method is that the ECM remains attached to the plate, maintaining its native three-dimensional structure, as there is hardly any manipulation, which allows the greatest number of components to be preserved and for it to remain as natural as possible. The plate can be used to grow cells directly on it; the cells must be added as quickly as possible.

**In** one particular embodiment, after collecting the NH₄OH solution, the plate can be gently washed to remove any remaining solution.

Washes can be done gently with water or PBS. Gently means removing the NH₄OH solution with the cell residues without dragging the ECM components.

**In** another particular embodiment, obtaining the ECM comprises the following steps:
1) de-cellularisation of the EPIC cell line with deposit number DSM ACC3373 attached to a culture plate using an NH₄OH solution for at least 5 minutes,
2) collecting and discarding the NH₄OH solution from the previous step,
3) dragging the gelatinous phase that forms on the plate after removing the NH₄OH solution from the plate, collecting it and washing it with distilled water and PBS to obtain the soluble fraction,
3) washing the bottom of the culture plate with distilled water to obtain the insoluble fraction,
where steps 1-3 must be performed in the order indicated.

The native three-dimensional structure of the ECM begins to degrade when the EPIC cells are removed. Some components detach from the structure faster than others, forming a gelatinous phase that can be collected. This is the soluble ECM phase. Next, to obtain the solid phase that is attached to the plate, it is necessary to wash intensely or even scrape to collect the insoluble ECM fraction that is attached to the culture plate.

The distilled water is preferably MiliQ. It is advisable for the water used to be cold to facilitate the separation of the insoluble fraction (ECM-IM or EPIC-IM) from the plate, i.e. at a temperature below 10°C.

**In** another particular embodiment, the cell line from which the ECM of the invention originates is cultured on a culture plate for a period of between 4 and 15 days, preferably between 6 and 10 days at a temperature of between 30°C and 40°C, preferably around 37°C and approximately 5% CO₂.

The appropriate culture medium for growing the EPIC cell line can be any culture medium suitable for growing a murine cell line *in vitro,* for example, Dulbecco's Modified Eagle Medium (DMEM), Roswell Park Memorial Institute (RPMI) and derivatives thereof. These media may contain antibiotics and supplements to promote cell growth, such as Fetal Bovine Serum (FBS). An expert in the field would be able to identify a suitable culture medium and, if necessary, modify it to promote the growth of the EPIC cell line using common general knowledge and standard techniques in the field.

**In** another particular embodiment, the cell line is kept in culture for a period of time between 1 day and 7 days after reaching a confluence of between 80% and 100%, preferably around 100%, the period of time being preferably between 2 days and 5 days.

The advantage of the aforementioned intervals is that the cell line can reach a high number of cells without contact inhibition problems, allowing it to generate a high amount of the ECM components responsible for its proliferative and proangiogenic characteristics.

Another additional object of the invention relates to the use or method of use of the murine extracellular matrix derived from the EPIC cell line, either alone or in combination with gelatine and/or other similar supports suitable for the culture of a cell line.

This use or method of use comprises bringing a cell line into contact with the extracellular matrix of the invention and optionally gelatine and/or other similar supports, which may also include a culture medium suitable for said cell line.

This achieves an increase in the proliferation and/or angiogenesis of the cell line compared to the normal levels of proliferation and/or angiogenesis of said cell line when cultured without the extracellular matrix of the invention.

Preferably, the culture of the cell line with the extracellular matrix of the invention is carried out *in vitro.*

In one particular embodiment, the insoluble fraction (ECM-IM or EPIC-IM) is used for the culture of cell lines.

In another particular embodiment, the soluble fraction (ECM-SM or EPIC-SM) is used for cell line culture.

In another particular embodiment, the murine extracellular matrix may be a mixture of the insoluble fraction (ECM-IM) and the soluble fraction (ECM-SM). That is, after isolating the ECM from the cell line from which it originates and separating the insoluble phase from the soluble phase, these can be recombined in different proportions, preferably 1:1 ECM-SM: ECM-IM, or from 0.1-0.9:1 ECM-SM: ECM-IM, or 1:0.1-0.9 ECM-SM: ECM-IM, or 1:0.5 ECM-SM: ECM-IM or 0.5:1 ECM-SM: ECM-IM.

### In another particular embodiment

In a particular embodiment, the concentration of the extracellular matrix and/or the soluble fraction (EPIC SM) and/or insoluble fraction (EPIC IM) is at least 0.05 µg/ml, preferably at least 0.5 µg/ml, more preferably at least 5 µg/ml, even more preferably at least 50 µg/ml, up to a maximum of 1000 µg/ml, or 500 µg/ml, or 200 µg/ml. The volume is the appropriate medium for culturing the cell line.

In another particular embodiment, the cell line that is brought into contact with the ECM of the invention may be of human origin, such as HUVEC or AC16 lines; or murine, such as HL-1 line. A person skilled in the art would identify the appropriate culture medium for growing the cell line(s) on which the ECM of the invention would be used, employing common general knowledge.

Another additional object of the invention relates to the murine extracellular matrix derived from the EPIC cell line for its use in cardiac conditions as a cardiac wall repair agent.

Each of the terms "comprising", "consisting essentially of," and "consisting of" may be substituted for either of the other two terms. The terms "a" or "an" may refer to one or a plurality of the elements being modified (e.g., "a reagent" may mean one or more reagents), unless it is contextually clear that it is described one of the elements or more than one. The term "approximately," as used herein, refers to a value within 10% of the underlying parameter (i.e., plus or minus 10%; for example, a weight of "approximately 100 grams" may include a weight between 90 grams and 110 grams). The use of the term "approximately" at the beginning of a list of values modifies each of the values (e.g., "approximately 1, 2, and 3" refers to "approximately 1, approximately 2, and approximately 3"). When a list of values is described, the list includes all intermediate values and all fractional values thereof (e.g., the list of values "80%, 85% or 90%" includes the intermediate value 86% and the fractional value 86.4%). When a list of values is followed by the term "or more", the term "or more" applies to each of the listed values (for example, the list "80%, 90%, 95% or more" or "80%, 90% or 95% or more" refers to "80% or more, 90% or more, or 95% or more"). When describing a list of values, the list includes all ranges between two of the listed values (e.g., the list of "80%, 90% or 95%" includes ranges of "80% to 90%", "80% to 95%" and "90% to 95%"). Below are some examples of how the technology is applied.

### Brief description of the figures

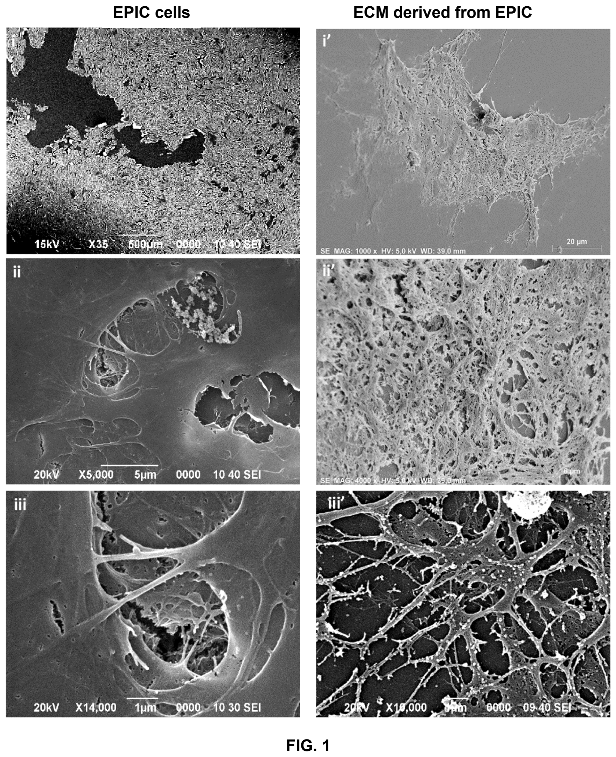

**Figure 1****.** Representative SEM (scanning electron microscope) images of EPIC cells at 35X (i), 5000X (ii) and 14000X (iii) magnification, and of the ECM obtained from EPIC-IM at 1,000X (i'), 4,000X (ii') and 10,000X (iii') magnification.

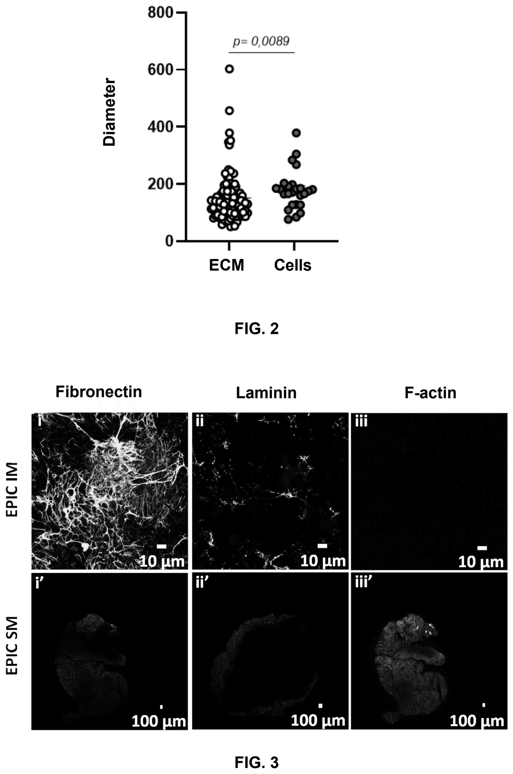

**Figure 2****.** Fibre diameters in EPIC-ECM (left column) and EPIC cell protrusions (right column) measured with ImageJ software using SEM images. Data from 6 biological replicates and 17 technical measurements each for the ECM samples, and 4 biological replicates with between 5 and 8 measurements each for the EPIC cells. Statistical analysis performed with the Mann Whitney test.
**Figure 3****.** Immunocytochemical analysis of fibronectin, laminin α1 subunit and F-actin in EPIC-IM (I, ii and iii) and EPIC-SM (i', ii' and iii'), respectively.

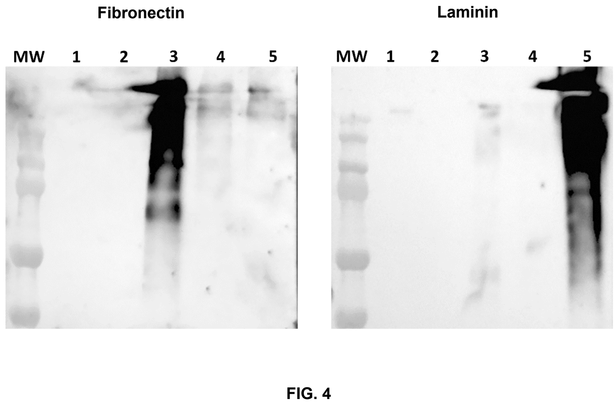

**Figure 4****.** Western Blot of lysates from EPIC cells (lane 1), EPIC IM cells (lane 2), EPIC SM (lane 3), ECM isolated from de-cellularised E17.5 embryonic hearts (lane 4) and Matrigel^{®} (lane 5) for fibronectin and laminin α1 subunit, MW: molecular weight of the ladder or marker.

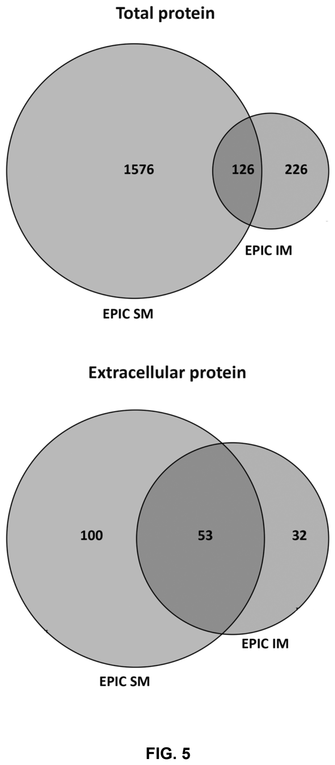

**Figure 5****.** Qualitative comparison of proteins isolated from EPIC IM and EPIC SM fractions and identified by label-free proteomic analysis. Venn diagram representation of the total proteins identified in EPIC IM and EPIC SM indicating the number of unique proteins in, at least, two biological replicates of EPIC IM (226 proteins) and EPIC SM (1,576 proteins), as well as the proteins found in common between both fractions (126 proteins). Venn diagram representation of extracellular proteins identified in EPIC IM and EPIC SM indicating the number of unique proteins in at least two biological replicates of EPIC IM (32 proteins) and EPIC SM (100 proteins) and the proteins common to both fractions (53 proteins).

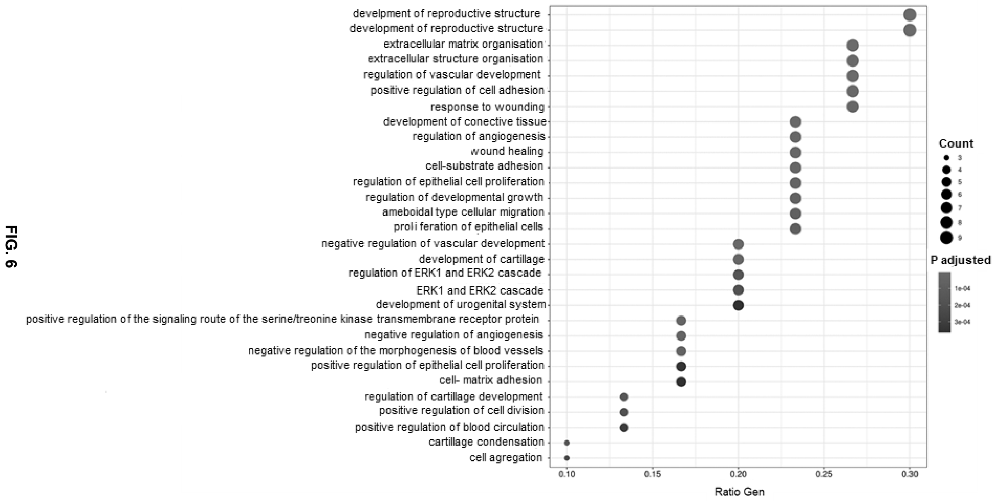

**Figure 6****.** Functional enrichment analysis of EPIC IM extracellular proteins. Biological processes (gene ontology analysis - GO terms) of EPIC IM extracellular proteins represented in a scatter plot. The most significant categories in descending order in the protein proportion categories. The x-axis represents the proportion of proteins in the analysed sample that is related to the specific GO indicated on the y-axis. The size of the dot represents the number of proteins associated with each functional category, and the colour of the dot corresponds to the adjusted p-value. In all cases, it is less than p<0.05, therefore statistically significant.

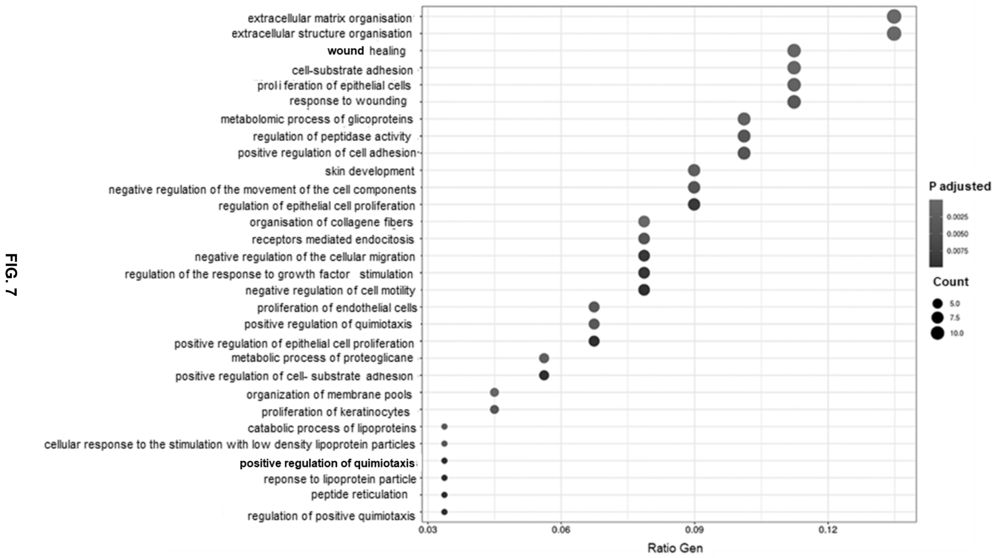

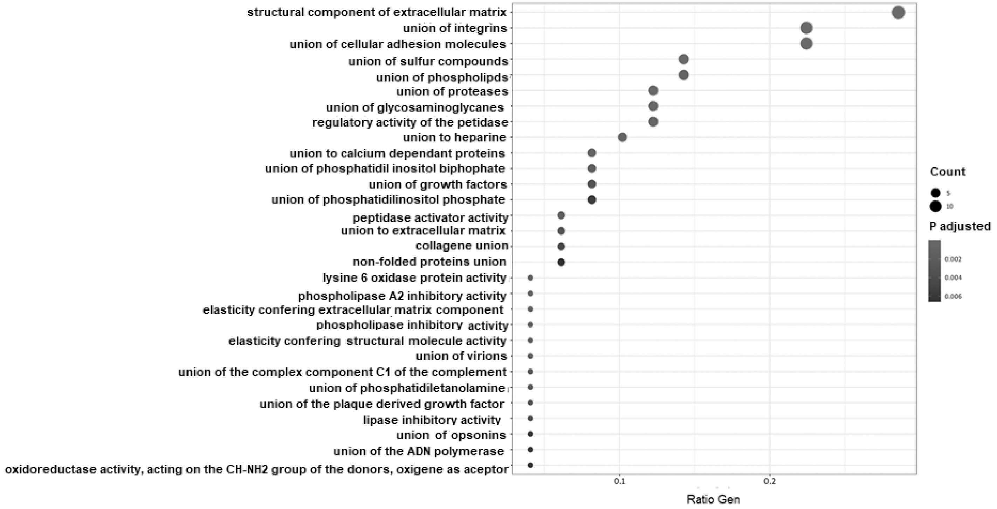

**Figure 7****.** Functional enrichment analysis of EPIC SM extracellular proteins. Biological processes (gene ontology analysis) of EPIC SM extracellular proteins represented in a scatter plot. The most highly significant categories in descending order in the protein proportion categories. The x-axis represents the proportion of proteins in the sample related to the specific GO category; the size of the dot represents the number of proteins associated with the functional category, and the colour of the dot corresponds to the adjusted p-value. In all cases, it is less than p<0.05, therefore statistically significant.

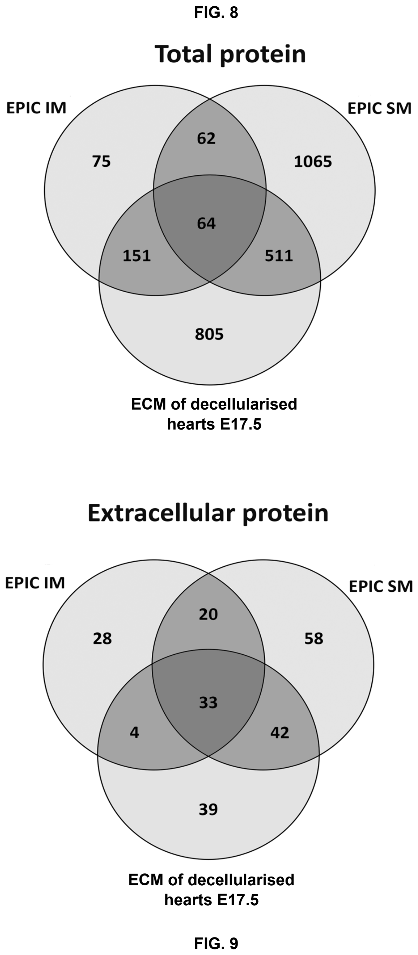

**Figure 8****.** Functional enrichment analysis of extracellular proteins common between EPIC IM and EPIC SM. The Y-axis represents the most significant gene ontology categories (GO terms) in descending order to which the identified proteins are associated. The x-axis represents the proportion of proteins and the size of the dot represents the number of proteins associated with the functional category, and the colour of the dot corresponds to the adjusted p-value. In all cases, it is less than p<0.05, therefore statistically significant.
**Figure 9****.** Qualitative comparison of ECM isolated from de-cellularised E17.5 murine embryonic hearts, the EPIC IM fraction and the EPIC SM fraction, identified by a label-free proteomic study. Venn diagram representation of the total and extracellular proteins identified in each of the samples, indicating the number of unique proteins in, at least, two replicates of EPIC IM (75 and 28 proteins, respectively), EPIC SM (1,065 and 58 proteins, respectively), E17. 5 (805 and 39 proteins, respectively) and proteins common only to the EPIC IM and SM fractions (62 and 20 proteins, respectively), EPIC IM and E17.5 hearts (151 and 4 proteins, respectively), EPIC SM and E17.5 hearts (511 and 42 proteins, respectively) and proteins common to all samples (64 and 33 proteins, respectively).

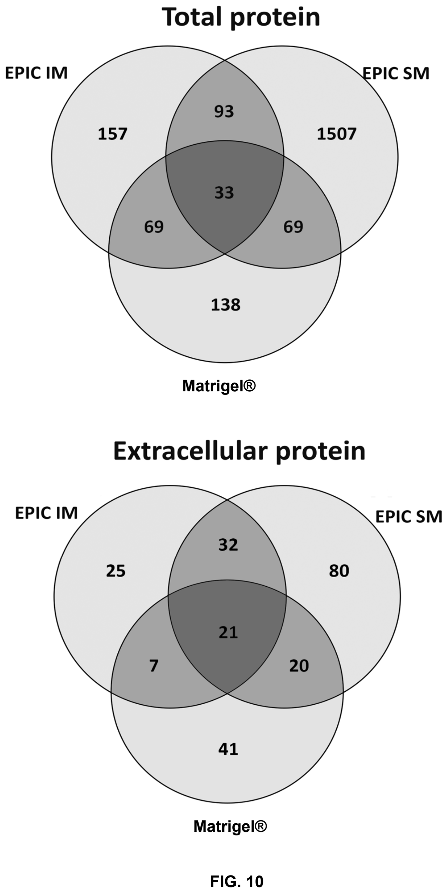

**Figure 10****.** Qualitative comparison of proteins isolated from Matrigel^{®}, the EPIC IM fraction and the EPIC SM fraction and identified by a label-free proteomic study. Venn diagram representation of total and extracellular proteins identified in the three samples, indicating the number of unique proteins in, at least, two replicates of EPIC IM (157 and 25 proteins, respectively), EPIC SM (1,507 and 80 proteins, respectively), Matrigel^{®} (138 and 41 proteins, respectively) and proteins common only to the EPIC IM and SM fractions (93 and 32 proteins, respectively), EPIC IM and Matrigel^{®} (69 and 7 proteins, respectively), EPIC SM and Matrigel^{®} (69 and 20 proteins, respectively) and proteins common to all samples (33 and 21 proteins, respectively).

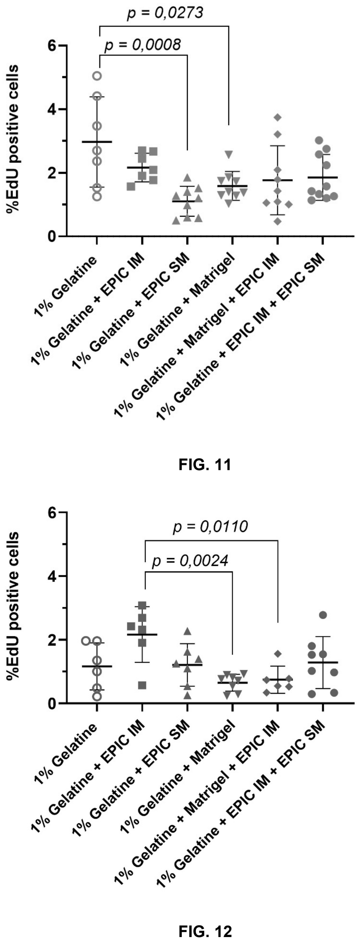

**Figure 11****.** Proliferation assay of HUVECs cultured for three days on surfaces coated with ECM derived from EPIC and Matrigel^{®}. Percentage of EdU incorporation in HUVECs incubated with 1% gelatine; 1% gelatine and 100 µg/mL EPIC IM; 1% gelatine and 100 µg/mL EPIC SM; 1% gelatine and 100 µg/mL Matrigel^{®}; 1% gelatine, 100 µg/mL Matrigel^{®} and 100 µg/mL EPIC IM; and 1% gelatine, 100 µg/mL EPIC IM and 100 µg/mL EPIC SM. Statistical analysis performed using two-way ANOVA with Tukey-Bonferroni corrections.
**Figure 12****.** Proliferation assay of HUVECs cultured for five days on surfaces coated with ECM derived from EPIC and Matrigel^{®}. Percentage of EdU incorporation in HUVECs incubated with 1% gelatine; 1% gelatine and 100 µg/mL EPIC IM; 1% gelatine and 100 µg/mL EPIC SM, 1% gelatine and 100 µg/mL Matrigel^{®}; 1% gelatine, 100 µg/mL Matrigel^{®} and 100 µg/mL EPIC IM; and 1% gelatine, 100 µg/mL EPIC IM and 100 µg/mL EPIC SM. Statistical analysis performed using two-way ANOVA with Tukey Bonferroni corrections.

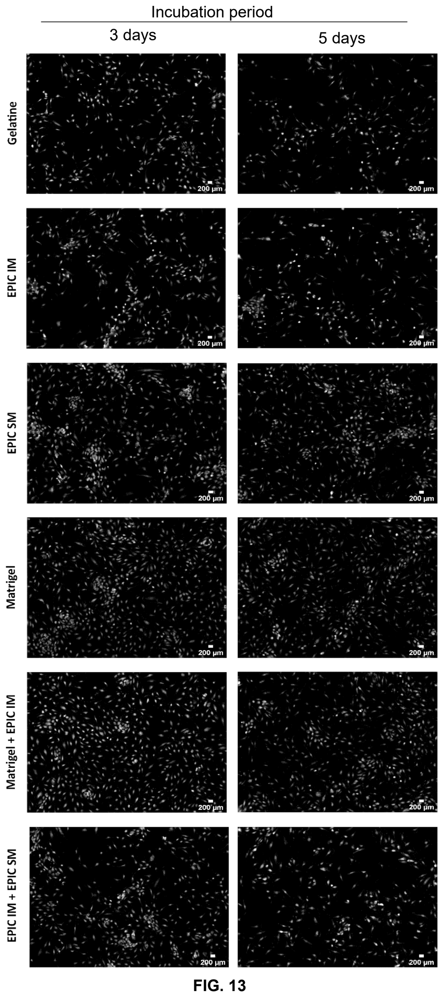

**Figure 13****.** Representative phase contrast images of HUVECs placed on surfaces coated with gelatine, gelatine with EPIC IM, gelatine with EPIC SM, gelatine with Matrigel^{®}, gelatine with EPIC IM and Matrigel^{®}, and gelatine with EPIC IM and EPIC SM. The scale of each image is 200 µm.

### Examples

### EXAMPLE 1

### Isolation of murine extracellular matrix from the EPIC cell line

For EPIC-ECM isolation, EPIC cells are seeded in monolayer on plastic cell culture flasks or plates, as well as on a glass slide, in cell culture medium containing 10% foetal bovine serum, DMEM without pyruvate, 1% L-glutamine and, optionally, antibiotics, e.g. 1% streptomycin/penicillin.

The cells are incubated for a period of seven days at 37 °C and 5% CO₂. To stimulate ECM deposition, the EPIC cell culture medium is refreshed every 24 hours from 70% cell confluence. EPIC is kept in culture for three days after reaching 100% confluence to obtain an ECM protein-enriched content.

Next, a de-cellularisation protocol is performed, adapted from Harris et al., 2018 (Harris, G.M., Raitman, I. and Schwarzbauer, J.E. (2018) 'Cell-derived decellularized extracellular matrices', Methods in Cell Biology, 143, pp. 97-114. doi:10.1016/bs.mcb.2017.08.007). Once the EPIC cultures are hyperconfluent at around 100% confluence, the cells are gently washed with PBS heated to 37 °C. The de-cellularisation is carried out by carefully pipetting 20 mM NH₄OH diluted in deionised water while avoiding abrupt shaking of the cell container. The cells are incubated for 5 minutes at room temperature while the container is carefully tilted to ensure lysis of all cells and allow them to detach. The recovered ammonium hydroxide solution containing cellular and extracellular components, hereinafter referred to as the soluble EPIC fraction (EPIC SM), and the extracellular elements were collected and washed first in MiliQ water and then in PBS. The fraction adhering to the bottom of the culture plate, hereinafter referred to as the insoluble EPIC fraction (EPIC IM), was carefully washed in cold MiliQ water.

Next, the EPIC SM fraction is collected in the fraction containing NH₄OH, while the EPIC IM fraction remains attached to the plastic or glass surface. Depending on their subsequent application, the EPIC fractions are fixed in paraformaldehyde or glutaraldehyde for IHC or SEM imaging, respectively, or denatured in a highly reducing Laemmli buffer (containing 100 mM DTT) for proteomic evaluation. For *in vitro* studies, the EPIC IM fraction is solubilised in 2M filtered at 0.2 µm urea and then washed and concentrated in sterile PBS by ultrafiltration (3 kDa). For the EPIC SM fraction, the sample is washed extensively in sterile MiliQ and finally in sterile PBS, in which it is homogenised.

### EXAMPLE 2

### EPIC IM presents a network in the form of a typical mesh and morphology of cell support

An exploratory analysis of the morphology of the EPIC cell line and ECM obtained from this cell line was carried out using SEM imaging, as its high resolution allows the sample to be analysed at the nanometric scale (Qu & Liu, Scanning, 2021; https://doi.org/10.1155/2021/4426254). This example shows the physical ultrastructure of the EPIC ECM after de-cellularisation and its collection.

Three days after having reached 100% confluence, the EPICs are de-cellularised as described above. The EPIC IM fraction of the ECM was washed in PBS (phosphate-buffered saline) at a pH of approximately 7.4 and fixed in 2% (v/v) glutaraldehyde diluted in PBS for 1 hour at room temperature, between 20°C and 28°C. For comparison, confluent plates containing EPIC cells were fixed without de-cellularisation in 2% glutaraldehyde and images were obtained. Both the EPIC samples and their ECM were washed again in PBS and incubated with increasing concentrations of ethanol for sample dehydration. Specifically, the samples were incubated for 15-minute periods in 30% ethanol, followed by 50%, 70%, 80%, 90%, 96%, and 100% ethanol, and then kept at 4°C until processed for scanning electron microscopy (SEM) imaging. The dehydrated samples were air-dried and deposited on an aluminium drum with double-sided conductive tape adhesive and coated with a thin layer of gold (300 Å) in a QOURUM Q 150R Sputtering (QOURUM). The samples were observed with the SEM JEOL JSM6490LV (JEOL) operating at 15 kV and a working distance of 10 mm. Images were taken at 35X, 5,000X, and 14,000X magnification for EPIC cells and at 1,000X, 4,000X, and 10,000X magnification for the EPIC IM fraction.

The scanning electron microscopy images show the fibrillar appearance of the EPIC cells and the EPIC IM fraction (Fig. 1) adhered to the surface of the slide. The smooth surface of EPIC cells contrasts with images of the EPIC IM fraction adhered to the glass slide, which presents a rough surface and a typical irregular pattern, assembling a mesh-like organisation typical of an ECM.

ImageJ 1.53i software was used to analyse the diameter of the fibres in the samples. Using the image size tool, the number of pixels/nanometres was evaluated, and the diameters were measured using the "Measure" tool from the analysis toolbar.

Seventeen fibre measurements were taken per photo, from a total of six photos of the EPIC IM SEM images, while from the EPIC cell images, five to eight measurements per photo were taken from four photos (Fig. 2). The diameter of the fibres was measured in the area of half length and the mean was calculated. Interestingly, while the fibres in the IM ECM fraction have a mean of 151 ± 8.5 nm, the EPIC cell protrusions, measured between cells, have a mean of 179.9 ± 13.91 nm. By applying a statistical t-test analysis, the means of the EPIC IM fibres and EPIC cell protrusions are significantly different, with a p-value of 0.0089 (Mann-Whitney test). These results demonstrate that the protein fractions isolated from EPICs are structurally ECM.

### EXAMPLE 3

### The ECM obtained from EPIC maintains laminin and fibronectin fibres in the soluble and insoluble fractions.

This example demonstrates the presence of fibronectin and laminin in both fractions, EPIC IM and EPIC SM. It also demonstrates the deposition pattern of fibronectin and laminin in EPIC IM. In addition, this example also demonstrates the purity level of the ECM fractions derived from EPIC using DNA and F-actin staining. The absence of DAPI signal confirms that there are no cells in the ECM sample.

To this end, immunocytochemistry was performed to identify the presence of intracellular and ECM-related proteins. After de-cellularisation, the surfaces containing EPIC IM were fixed with 4% paraformaldehyde (PFA) for 10 minutes at room temperature, while the wells containing EPIC cells were fixed with 4% PFA for 20 minutes at room temperature. The ECM obtained from EPIC was fixed in 4% PFA for 30 minutes after washing by centrifugation in PBS. The fixed ECM fractions and cells were washed three times in PBS and blocked with SBT (10% horse serum, 1.5% BSA, and 0.5% Triton X-100 in TPBS) for 1 hour at room temperature. The two EPIC-ECM fractions and cells were washed with PBS and incubated overnight at 4°C with 1:100 polyclonal rabbit anti-fibronectin (F3648; Sigma) or 1:200 monoclonal anti-laminin α1 subunit (L9393; Sigma), both diluted in SBT. Samples were washed in PBS and then incubated with 1:2000 4',6-diamidino-2-phenylindole (DAPI) and 1:200 anti-rabbit AF647 (Jackson Immuno Research) (antibody:PBS) for 1 hour at room temperature. Finally, the slides were washed three times in PBS. For F-actin staining, samples were incubated with 0.6 µM Phalloidin Atto 488 (A12379; Thermo Fisher) and 1:2500 4',6-diamidino-2-phenylindole (DAPI) for 20 min on a shaker at room temperature in the dark.

Samples were analysed using Leica SP5 HyD confocal microscope. The images were processed using ImageJ 1.53i software, applying the maximum intensity of the stacked images from the "Z Project" tool.

The partial components of EPIC IM and SM are identified by laminin and fibronectin (Fig. 3). In EPIC IM, it is observed that fibronectin appears to maintain its native structure, only more compact after chemical de-cellularisation. In addition, some empty areas with laminin α1 deposits are observed around gaps where, according to their morphology, there were cells before. As for EPIC SM, fibronectin residues are observed throughout the mounted sample.

Laminin α1 is also scarcely distributed in EPIC SM, similar to fibronectin in the same ECM fraction.

To evaluate the effect of our de-cellularisation process on intracellular proteins and their binding to the matrix, F-actin, an actin polymer abundant in the cytoskeleton of eukaryotic cells, was labelled. No F-actin staining was found, indicating that there are no intact cells and the intracellular cytoskeletal protein content is zero. In addition, EPIC SM has a high amount of DNA contamination, while in EPIC IM a weak DAPI signal bound to fibronectin or laminin fibres can be found.

The presence of laminin and fibronectin was also confirmed by Western Blot. In this case, 8% polyacrylamide gels were prepared in which 10 µg of EPIC cell lysate, EPIC IM and EPIC SM were prepared in 2X concentrated loading buffer, containing 0.03 M Tris-HCl pH 6.8, 1% SDS, 2.5% (v/v) β-mercaptoethanol, 0.025% (v/v) bromophenol blue, and 10% (v/v) glycerol. After boiling the protein samples at 95°C for 5 minutes, the samples and ladder were loaded and run at 100 V for 10 min to allow the samples to enter the gel homogeneously. The proteins were then separated in SDS-polyacrylamide gel with bis-acrylamide concentrations between 8 and 12% at 200 V for an average of 90 minutes. The separated proteins were transferred to nitrocellulose membranes (Protan, 0.45 µm) using the Mini Trans-Blot Cell system coupled to the PowerPac HC high-current power supply (BIO-RAD) in Laemmli transfer buffer. Transfers were performed at 350 A for 3 hours, maintaining the Cell system at 4 °C during transfer. Any person skilled in the art would know how to vary the experimental conditions to obtain the same result using standard protocols in the area.

After transfer, the membranes were washed in TBST (4% (m/v) NaCl, 0.1% KCI, 12.5% (v/v) 1 M Tris HCl pH 7.5 and 0.01% Tween 20 (Sigma) in distilled water) for 10 min to remove Laemmli transfer buffer residues. To confirm the transfer of proteins to the nitrocellulose membrane, a general protein stain was performed using Ponceau red with 5% (w/v) dye in 0.1% (v/v) acetic acid. The membranes were then washed in TBST to remove staining residues. The membranes were blocked in 5% (w/v) skim milk in TBST (blocking buffer) for 1 hour at room temperature on a shaker and then washed in TBST.

The membranes were then incubated with a primary anti-laminin α1 subunit monoclonal antibody (1:1000; L9393, Sigma) or with an anti-fibronectin polyclonal antibody (1:1000; F3648, Sigma) in blocking buffer overnight at 4 °C on a shaker. After incubation, the membranes were washed in TBST and incubated with a secondary anti-rabbit IgG antibody in TBST (1:10,000; A5278, Sigma) for 1 hour at room temperature. Finally, the membranes were washed in TBST and incubated for 5 minutes in the dark with SuperSignal^{®} West Pico PLUS chemiluminescent substrate reagent (ThermoFisher) according to the manufacturer's instructions. The signal was revealed using Chemidoc XRS+ ultraviolet imaging (BIO-RAD), and the images were processed with Image Lab software (BIO-RAD).

The EPIC-SM fraction shows the highest intensity in the presence of fibronectin, even in greater proportion than the total extract from an embryonic mouse heart or commercial Matrigel^{®}. This demonstrates that the EPIC-SM fraction is enriched in ECM proteins. However, in the EPIC-IM fraction, whose physical ECM structure was demonstrated with SEM (Fig. 1), only a thin band is visible at the top of the membrane, indicating that there are clear differences between the two fractions in terms of protein composition (Fig. 4). In the case of laminin, EPIC SM shows a signal at the top of the membrane. No bands were detected in EPIC IM. However, Matrigel^{®} is enriched in laminin compared to the other samples included in the experiment. As for the EPIC cell lysate, neither fibronectin nor laminin was detected due to their relatively low abundance in the solution.

### EXAMPLE 4

### Proteomic analysis of ECM obtained from the EPIC cell line using liquid chromatography-tandem mass spectrometry

An ascending LC-MS/MS (Liquid Chromatography-Tandem Mass Spectrometry) was performed to identify proteins in both fractions of the ECM derived from EPIC. These results provide a qualitative correlation of the number of proteins present in the EPIC IM and SM fractions.

Both ECM fractions were homogenised in SDS-PAGE buffer composed of 6% (v/v) glycerol, 2% (m/v) SDS, 100 mM dithiothreitol (DTT) and 0.05 M Tris-HCl pH 6.8 at 95 °C and stored at -80 °C. Due to its insoluble nature, a high concentration of DTT was applied to improve the efficiency of EPIC-IM extraction. The insoluble fraction dissolved in Laemmli buffer (LB) was thoroughly scraped using cell scrapers. For EPIC-SM homogenisation, the samples were boiled for 15 minutes and vortexed intermittently every 2 minutes. Subsequently, the samples were sonicated in an ultrasonic bath for 5 minutes and centrifuged at 14,000 g for 5 minutes. The supernatant was used for unlabelled mass spectrometry analysis.

To remove LC-MS-incompatible components, EPIC IM and EPIC SM supernatants underwent gel-assisted proteolysis by mixing 45 µL of sample with 14 µL of 40% acrylamide. Then, 2.5 µL of 10% ammonium persulphate and 1 µL of TEMED were quickly added and incubated for 20 minutes. The gels were cut into 1-2 mm cubes and treated with 50% acetonitrile (ACN)/25 mM ammonium bicarbonate. The samples were dehydrated and dried with ACN and then reduced with 10 mM DTT in 50 mM ammonium bicarbonate for 30 minutes at 56 °C. Cysteine residues were carbamidomethylated with 55 mM iodoacetamide in 50 mM ammonium bicarbonate for 20 minutes at room temperature and in the dark. The gel fractions were dehydrated and the proteins were digested by rehydrating the gel fractions in 10 ng/µL trypsin overnight at 30 °C. The peptides were extracted with 0.1% ACN/formic acid (FA) for 30 min at room temperature. The samples were dried with SpeedVac to remove residual ACN and ammonium bicarbonate, dissolved in 0.1% FA, treated with ultrasound for 3 min, and centrifuged at 13,000 g for 5 min. Next, the samples were purified and concentrated using C18 ZipTip according to the manufacturer's instructions.

The purified samples were injected into a UHPLC Easy-nLC 1200 coupled to a Q Exactive HF-X Hybrid Quadrupole-Orbitrap mass spectrometer (ThermoFisher Scientific). The software versions used for data acquisition and manipulation were Tune 2.9 and Xcalibur4.1.31.9. The mobile phases of the HPLC were as follows: solvent A consisted of 0.1% FA (in water) and solvent B consisted of FA/ACN (0.1%/80%). Using a thermostated automatic injector, 1 µL (corresponding to 100 ng) of the peptide samples was loaded onto an Acclaim PepMap 100 precolumn, 75 µm x 2 cm, C18, 3 µm, 100 A (ThermoFisher Scientific) at a flow rate of 20 µL/min and eluted through a 50 cm PepMap RSLC C18, 2 µm, 100 A, 75 µm x 50 cm analytical column (ThermoFisher Scientific). The peptides were eluted from the analytical column with a 120 min gradient from 5% to 20% solvent B, followed by a 5 min gradient from 20% to 32% solvent B and finally to 95% solvent B for 10 min before rebalancing with 5% solvent B at a constant flow rate of 300 nL/min. LTQ Velos ESI Positive Ion Calibration Solution (Pierce, IL, USA) was used to externally calibrate the instrument prior to sample analysis, and internal calibration was performed using the polysiloxane ion signal at m/z 445.120024 from ambient air. MS1 scans were performed in the m/z 375-1,600 range with a resolution of 120,000. Using a data-dependent acquisition mode, the 15 strongest precursor ions from all precursor ions with a charge of +2 to +5 within a 1.2 m/z window were isolated and fragmented to obtain the corresponding MS² spectra. The fragmentation ions were generated in a high energy collisional dissociation (HCD) cell with a first mass fixed at 110 m/z and detected in an orbitrap mass analyser with a resolution of 30,000. The dynamic exclusion for the selected ions was 30 s. The maximum ion accumulation time allowed in MS and MS² mode was 50 ms and 70 ms, respectively. Automatic gain control was used to prevent ion trap overfilling and was set at 3x10⁶ ions and 2x10⁵ ions for a complete MS and MS2 scan, respectively.

Searches of the MS/MS² spectra were performed in the SwissProt *Mus musculus* protein database version 2017.10.25 (25,097 sequences). The acquired raw data was analysed in the Proteome Discoverer 2.2 platform (Thermo Fisher Scientific) with the Sequest HT search engine using mass tolerances of 10 ppm and 0.02 Da for precursor ions and fragment ions, respectively. Two tryptic cleavages were allowed to be missed. Methionine oxidation and N-terminal acetylation were set as variable modifications, while carbamoyl methylation of cysteine residues was set as a fixed modification.

Proteins found under different experimental conditions were compared at a qualitative level (presence or absence). The FDR (*False Discovery Rate*) for consecutive protein and peptide assignments was determined using the *Percolator* software package, which is based on a target decoy approach using an inverted protein database as a decoy, imposing a strict 1% FDR limit. The results were filtered to accept only those proteins with at least two peptide sequences.

The protein lists were enriched in terms of Gene Ontology (GO) using the *clusters_to* _*enrichments*.*R de ExpHunterSuite* script (González Gayte et al., Genomics and Computational Biology, 2017 - DOI: 10.18547/gcb.2017.vol3.iss3.e31). This script uses over-representation analysis (ORA). This method takes a group of significant proteins and performs a Fisher's exact test for each GO term. The P-value of the Fisher's exact test was corrected using the Benjamini-Hochberg procedure. GO terms with an adjusted P-value lower than 0.05 were discarded.

In this case, the EPIC IM extracellular protein lists were compared with EPIC SM to analyse the differences in protein content between the two fractions. In EPIC IM, a total of 404 proteins were identified in three biological replicates, of which 352 were common in at least two replicates. In EPIC SM, 1,707 proteins were detected, of which 1,702 were present in two of the three replicates. The total proteins from EPIC IM and SM share 126 proteins (Fig. 5). Due to our interest in ECM proteins, the "extracellular proteins" filter was applied for further analysis. Here, the EPIC IM replicates contain 88 extracellular proteins, 85 of which were identified in at least two replicates, while 153 extracellular proteins were detected in EPIC SM. Of these, 53 extracellular proteins were shared with the EPIC IM fraction (Fig. 5 and Table 1). Thus, EPIC SM presents 100 unique extracellular proteins, while EPIC IM presents 32 unique proteins (Table 2).

**Table 1. Proteins common to EPIC IM and EPIC SM.**

| **UniProt ID** | **Name** | **UniProt ID** | **Name** |
|---|---|---|---|
| 008573 | Lgals9 | P39876 | Timp3 |
| 088569 | Hnrnpa2b1 | P48759 | Ptx3 |
| P01029 | C4b | P55065 | Pltp |
| P01887 | B2m | P62960 | Ybx1 |
| P01902 | H2-K1 | P62962 | Pfn1 |
| P02468 | Lamc1 | P82198 | TGF-βI |
| P02469 | Lamb1 | P97792-1 | Cxadr isoform 1 |
| P06745 | Gpi1 | Q01149 | Col1a2 |
| P07356 | Anxa2 | Q05793 | Hspg2 |
| P08113 | Hsp90b1 | Q08879 | Fbln1 |
| P10107 | Anxa 1 | Q61001 | Lama5 |
| P10649 | Gstm1 | Q61292 | Lamb2 |
| P11087-1 | Isoform 1 of Col1a1 | Q61398 | Pcolce |
| P11152 | Lpl | Q61703 | Itih2 |
| P11276 | Fn1 | Q62351 | Tfrc |
| P11499 | Hsp90ab1 | Q8CG19-1 | Long isoform of Ltbp1 |
| P13020-1 | Isoform 1 of Gsn | Q8R2G6 | Ccdc80 |
| P16110 | Lgals3 | Q8VDD5 | Myh9 |
| P17742 | Ppia | Q924C6 | Loxl4 |
| P18760 | Cfl1 | Q99K41 | Emilin1 |
| P19788 | Mgp | Q9CYL5 | Glipr2 |
| P20029 | Hspa5 | Q9D1D6 | Cthrc1 |
| P21956-1 | Isoform 1 of Mfge8 | Q9D6X6 | Prss23 |
| P27773 | Pdia3 | Q9R118 | Htra1 |
| P28301 | Lox | Q9WU78 | Pdcd6ip |
| P35441 | Thbs1 | Q9WVJ9 | Efemp2 |
| P37889 | Fbin2 | | |

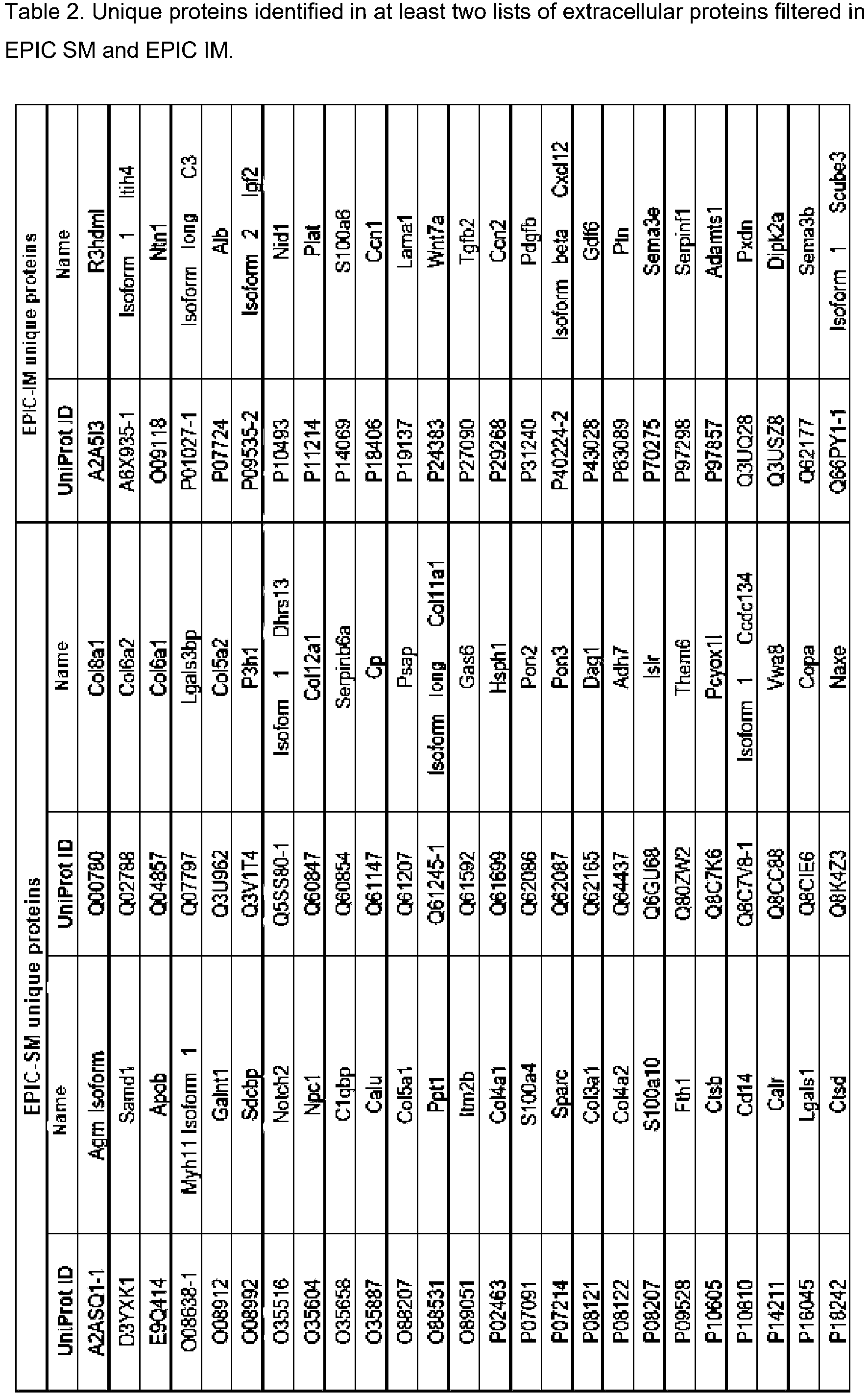

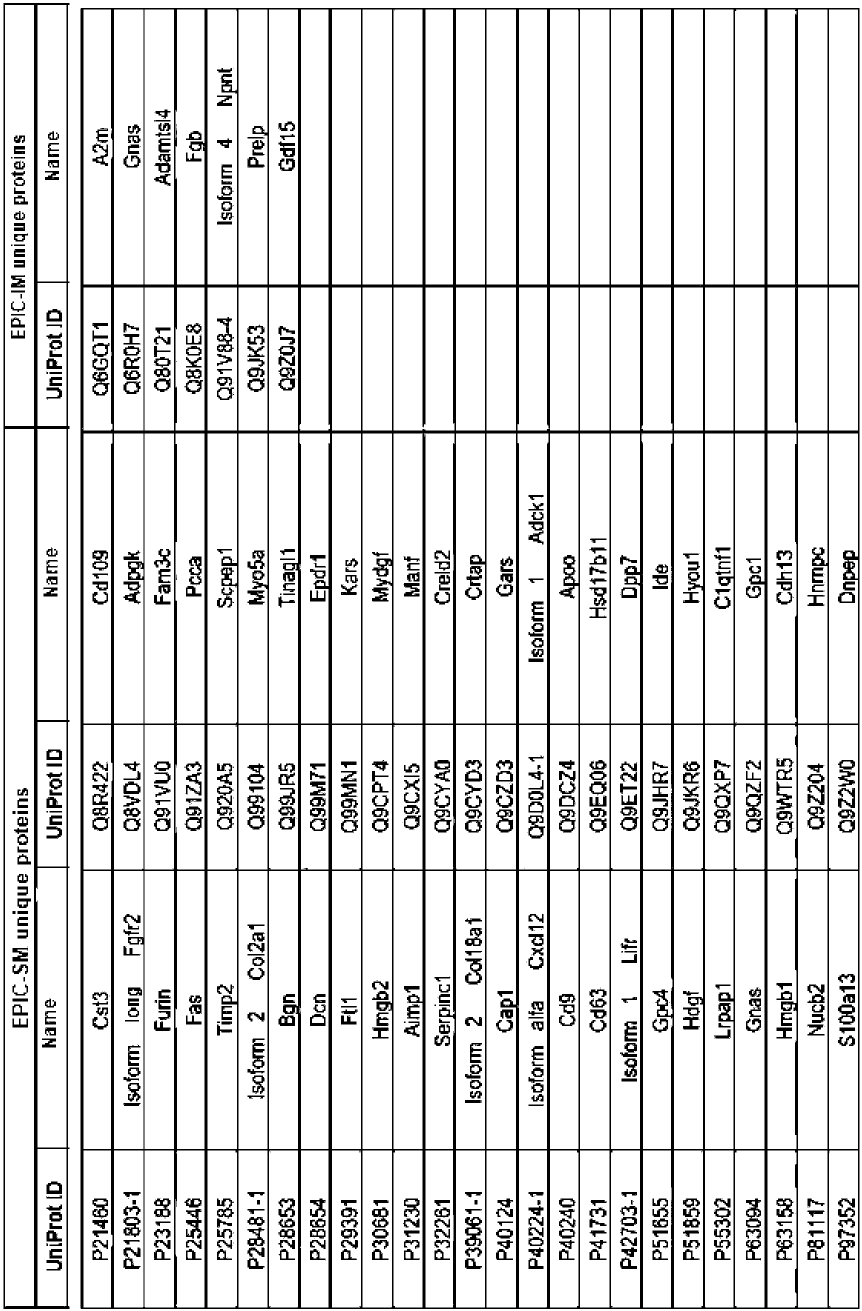

### EXAMPLE 4

### EPIC IM and EPIC SM share proteins associated with similar biological functions

By *in silico* analysis, the association of proteins exclusive to EPIC_IM_extracellular with gene ontology terms (*GO terms*) was established (Fig. 6). "GO:0030198 - extracellular matrix organisation" and "GO:0043062 - extracellular structure organisation" are the next terms that also show an adjusted p-value of 2.35 x 10⁻⁶, followed by "GO:1901342 - regulation of vascular development" (adjusted p-value = 3.67 x10⁻⁶), "GO:0045785 - positive regulation of cell adhesion" (adjusted p-value = 1.08 x10⁻⁵ ) and "GO:0009611 - response to wounding" (adjusted p-value = 2.22 x10⁻⁵), with a protein ratio, defined as the proportion of significant proteins found in the functional category, of 0.267. In addition, "GO:0048608 - development of reproductive structure" and "GO:0061458 - development of reproductive system" are processes that also show an adjusted p-value of 2.35 x10⁻⁶, equal to the previous GO annotation related to ECM. It is important to note that the same protein may be included in different GO annotations and contribute to enhancing a particular term. Terms related to proliferation and development are also represented with higher adjusted p-values, such as "GO:0045765 - regulation of angiogenesis" (adjusted p-value = 2.22 x10⁻⁵), "GO:0050678 - regulation of epithelial cell proliferation" (adjusted p-value = 3.79 x 10⁻⁵), "GO:0048638 - regulation of developmental growth" (adjusted p-value = 5.96 x 10⁻⁵) and "GO:0050673 - epithelial cell proliferation" (adjusted p-value = 8.48 x 10⁻⁵), presenting a protein ratio of 0.23, and "GO:0070372 -regulation of ERK1 and ERK2 cascade" (adjusted p-value = 1.61 x 10⁻⁴) and "GO:0070371 - ERK1 and ERK2 cascade" (adjusted p-value = 1.93 x 10⁻⁴), among others, with a protein ratio of 0.2.

With regard to the unique GO terms related to the biological processes of EPIC_SM_extracellular proteins (Fig. 7), "GO:0030198 - organisation of extracellular matrix" and "GO:0043062 - organisation of extracellular structure" are GO terms that show an adjusted p-value of 1.28 x 10⁻⁶ and a protein ratio of 0.134. In this case, these terms correspond to a lower protein ratio than that of EPIC_IM_ext (0.134 vs. 0.267, respectively), indicating that, although these terms are the most representative of the unique extracellular proteins found in EPIC_SM_extracellular, in EPIC_IM_extracellular they are the most representative proteins within its extracellular protein content.

The GO terms described in EPIC_IM_extracellular, such as "GO:0042060 - wound healing" (adjusted p-value = 3.86 x 10⁻⁴), "GO:0031589 - cell-substrate adhesion" (adjusted p-value = 3.86 x10⁻⁴), "GO:0050673 - epithelial cell proliferation" (adjusted p-value = 1.73 x10⁻³) and "GO:0009611 - response to wounding" (adjusted p-value = 3.45 x10⁻³), are also found in EPIC_SM_extracellular. These GO terms have a higher proportion of proteins in EPIC_IM_extracellular than in EPIC_SM_extracellular. Therefore, the p-values shown and the proportions of proteins associated with extracellular of wound and vascular development GO terms are better in EPIC_IM_extracellular than in EPIC_SM_extracellular. This analysis indicates greater matrix support and a possible reparative role for EPIC IM compared to the EPIC SM fraction.

The functional enrichments of the extracellular proteins unique to each fraction reveal similar GO annotations in the biological processes of ECM-related terms, such as wound healing, epithelial cell regulation, and those related to vasculogenic processes. These results verify the embryonic epicardial nature of the cells of origin.

### EXAMPLE 5

### Composition and abundance of ECM derived from EPICs

In this example, the proteins secreted by EPIC-ECM are presented and their relative abundance in EPIC IM and EPIC SM is described. The relative abundances of the sample replicates were prepared by calculating the abundance of a protein relative to the total abundance per condition. In detail, the means of the abundances of each protein were calculated, divided by the total protein abundance, and multiplied by 100 to obtain a percentage of the relative abundance of each protein in solution. The total abundance was calculated from the sum of the abundances per replicate, and then an average of the total abundance per replicate was performed to obtain the total abundance per condition. Next, a list of proteins and their respective abundances was filtered in the "cell component" tab for the term "extracellular". For the ECM analysis, only extracellular proteins were considered.

In EPIC IM, the most abundant extracellular protein is serine protease HTRA1 (Q9R118) with 15.21% abundance in solution, while it represents only 0.01% of extracellular proteins in EPIC SM. Whereas in EPIC SM fibronectin (P11276) is the most abundant extracellular protein in solution (40.43%), in EPIC IM shows 3.36% of this protein in solution (Table 3), which verifies the results obtained by WB (Fig. 4).

**Table 3. Relative abundance of extracellular proteins in the total protein solution of EPIC IM and EPIC SM.**

| | Relative abundance over total protein content | | | Relative abundance over total protein content | |
|---|---|---|---|---|---|
| UniProt ID | EPIC IM | EPIC SM | UniProt ID | EPIC IM | EPIC SM |
| Q9R118 | 15.21% | 0.01% | P63094 | -- | 0.14 |
| P40224-2 | 5.26% | -- | P30681 | -- | 0.13% |
| P11276 | 3.36% | 40.43% | Q9JKR6 | -- | 0.13 |
| Q99K41 | 3.01% | 0.78 | Q9QZF2 | -- | 0.13 |
| P19137 | 2.76% | -- | P08121 | -- | 0.12% |
| P07356 | 2.44% | 2.55% | P08207 | -- | 0.09 |
| P19788 | 1.86 | 0.01% | P29391 | -- | 0.09 |
| P10493 | 1.78% | -- | P18242 | -- | 0.08% |
| P02468 | 1.59% | 0.48% | Q91ZA3 | -- | 0.08 |
| Q9R001 | 1.19% | -- | Q8VDL4 | -- | 0.07% |
| P39876 | 1.01 | 0.01% | P51655 | -- | 0.07 |
| Q9Z0J7 | 0.90% | -- | O35658 | -- | 0.06% |
| P02469 | 0.69% | -- | Q61207 | -- | 0.06% |
| P11087-1 | 0.66 | 1.64% | P09528 | -- | 0.05 |
| Q9D6X6 | 0.64 | 0.00% | Q8CIE6 | -- | 0.05 |
| P18406 | 0.59 | -- | O35887 | -- | 0.05% |
| Q8CG19-1 | 0.57 | 0.16% | Q99MN1 | -- | 0.05 |
| P63089 | 0.50 | -- | Q9DCZ4 | -- | 0.05% |
| P10649 | 0.46 | 0.02 | P42703-1 | -- | 0.05 |
| Q8VDD5 | 0.45 | 19.76% | P28653 | -- | 0.04% |
| P62960 | 0.31% | 0.01 | Q8R422 | -- | 0.04 |
| P82198 | 0.24 | 0.01% | 089051 | -- | 0.04 |
| Q80T21 | 0.22 | -- | Q61147 | -- | 0.04 |
| P10107 | 0.22% | 0.01% | P07214 | -- | 0.04 |
| Q05793 | 0.19 | 1.41% | O88207 | -- | 0.04% |
| P21956-1 | 0.18 | 0.28 | Q04857 | -- | 0.04 |
| P16110 | 0.15 | 0.13% | P41731 | -- | 0.04 |
| P70275 | 0.15% | -- | Q02788 | -- | 0.04% |
| P35441 | 0.14% | 0.30% | Q6GU68 | -- | 0.03 |
| P01027-1 | 0.13% | -- | Q8K4Z3 | -- | 0.02% |
| Q9WVJ9 | 0.13% | 0.00% | P63158 | -- | 0.02 |
| Q9WU78 | 0.11 | 0.03% | Q9EQ06 | -- | 0.02 |
| O08573 | 0.11 | 0.01% | Q99J R5 | -- | 0.02 |
| Q8R2G6 | 0.11 | 0.01% | Q9CZD3 | -- | 0.02 |
| O88569 | 0.10 | 0.10 | E9Q414 | -- | 0.02 |
| P28301 | 0.10 | 0.01% | Q3U962 | -- | 0.02 |
| P29268 | 0.10% | -- | Q9CPT4 | -- | 0.02% |
| P43028 | 0.10% | -- | Q07797 | -- | 0.02% |
| P37889 | 0.10% | 0.12% | P40240 | -- | 0.02 |
| 009118 | 0.09% | | P51859 | -- | 0.02 |
| P48759 | 0.08 | 0.01 | 035516 | -- | 0.02 |
| P11499 | 0.08 | 0.20 | P39061-1 | -- | 0.02 |
| P07724 | 0.07 | | P07091 | -- | 0.01 |
| Q62351 | 0.07 | 0.14% | Q9JHR7 | -- | 0.01 |
| Q61001 | 0.07 | 1.00 | P10605 | -- | 0.01 |
| Q3USZ8 | 0.06% | -- | P40224-1 | -- | 0.01% |
| P97857 | 0.06% | -- | Q9Z2W0 | -- | 0.01% |
| P13020-1 | 0.05% | 0.16% | Q9DOL4-1 | -- | 0.01 |
| Q61703 | 0.05 | 0.01 | O35604 | -- | 0.01 |
| P08113 | 0.05 | 2.92% | P40124 | -- | 0.01% |
| P20029 | 0.04% | 1.94% | Q9CXI5 | -- | 0.01 |
| A6X935-1 | 0.04% | -- | Q61699 | -- | 0.01% |
| P06745 | 0.04% | 0.02% | Q3V1T4 | -- | 0.01 |
| P11152 | 0.04% | 0.01 | P55302 | -- | 0.01 |
| Q9CYL5 | 0.04 | 0.03 | P08122 | -- | 0.01 |
| P17742 | 0.04 | 0.18 | Q5SS80-1 | -- | 0.01 |
| P62962 | 0.04 | 0.04% | Q80ZW2 | -- | 0.01 |
| P01902 | 0.04% | 0.14% | Q62087 | -- | 0.01 |
| Q91V88-4 | 0.04% | -- | Q8C7V8-1 | -- | 0.01% |
| Q6R0H7 | 0.04% | -- | Q920A5 | -- | 0.01% |
| P55065 | 0.03% | 0.05 | Q62165 | -- | 0.01 |
| P97298 | 0.03% | -- | Q62086 | -- | 0.01% |
| P09535-2 | 0.03% | -- | Q9Z204 | -- | 0.01% |
| Q3UQ28 | 0.02% | -- | Q9CYA0 | -- | 0.01% |
| P16045 | 0.02% | 0.07% | Q9WTR5 | -- | 0.01 |
| Q62177 | 0.02% | -- | Q00780 | -- | 0.01% |
| P24383 | 0.02% | -- | P25785 | -- | 0.01% |
| Q66PY1-1 | 0.02% | -- | Q9CYD3 | -- | 0.01% |
| Q61292 | 0.02% | 0.27 | Q64437 | -- | 0.01 |
| P27090 | 0.01% | -- | P28481-1 | -- | 0.01% |
| P27773 | 0.01% | 1.62 | Q61592 | -- | 0.01 |
| Q08879 | 0.01 | 0.01% | Q99M71 | -- | 0.01% |
| A2A5I13 | 0.01% | -- | 008912 | -- | 0.00% |
| Q9JK53 | 0.01% | -- | P25446 | -- | 0.00% |
| P18760 | 0.01% | 0.04% | P31230 | -- | 0.00 |
| O08665 | 0.01% | -- | O08992 | -- | 0.00% |
| P97792-1 | 0.01% | 0.02% | A2ASQ1-1 | -- | 0.00 |
| Q61398 | 0.01 | 0.08 | 088531 | -- | 0.00 |
| P31240 | 0.01% | -- | P28654 | -- | 0.00% |
| Q8K0E8 | 0.01% | -- | P97352 | -- | 0.00% |
| Q6GQT1 | 0.01% | -- | P23188 | -- | 0.00% |
| P11214 | 0.01% | -- | Q60854 | -- | 0.00% |
| P01029 | 0.00% | 0.05% | P02463 | -- | 0.00 |
| Q01149 | 0.00 | 0.25% | P32261 | -- | 0.00 |
| Q924C6 | 0.00 | 0.01% | D3YXK1 | -- | 0.00 |
| Q9D1D6 | 0.00 | 0.02% | Q61245-1 | -- | 0.00 |
| P01887 | 0.00 | 0.01% | P81117 | -- | 0.00 |
| P14211 | -- | 0.70% | P21460 | -- | 0.00% |
| 008638-1 | -- | 0.70% | P10810 | -- | 0.00% |
| Q8C7K6 | -- | 0.30% | Q91VU0 | -- | 0.00% |
| Q60847 | -- | 0.22% | Q9QXP7 | -- | 0.00% |
| Q8CC88 | -- | 0.18% | P21803-1 | -- | 0.00 |
| Q99104 | -- | 0.16% | Q9ET22 | -- | 0.00% |

### EXAMPLE 6

### The ECM obtained from EPIC shows similarities with decellularised embryonic hearts

To evaluate the contribution of the epicardial ECM to the embryonic heart ECM, ECM derived from EPIC was compared with ECM from E17.5 embryonic hearts decellularised with NH₄OH. A preliminary analysis shows that the ECM derived from EPIC contains proteins in common with the MEC of murine hearts of embryonic day 17.5 (E17.5).

The workflow of the *in silico* analysis used was as in example 4. A total of 1,531 proteins were identified in the ECM from E17.5 hearts. Sixty-four proteins are common to the E17.5 cardiac ECM, EPIC IM, and EPIC SM, while 151 proteins are shared exclusively between EPIC IM and the E17.5 cardiac ECM, and another 511 proteins are shared only between the E17.5 cardiac ECM and EPIC SM.

When we apply the "extracellular proteins" filter, the number is reduced to 118 extracellular proteins identified in the ECM of embryonic hearts. Of these, the ECM derived from EPIC shares 79 extracellular proteins with de-cellularised E17.5 hearts.

Specifically, de-cellularised hearts, the EPIC SM fraction, and the EPIC IM fraction share 33 extracellular proteins, as indicated in Table 4 and represented in Fig. 9. As for extracellular proteins shared exclusively between EPIC IM and E17.5 cardiac ECM, four proteins are identified. As for the exclusive proteins common to EPIC SM and E17.5 cardiac ECM, 42 proteins were identified (Table 4).

**Table 4. Proteins common to the ECM of de-cellularised E17.5 murine hearts and EPIC IM (4 proteins) and EPIC SM (42 proteins) and proteins common to all conditions (33 proteins).**

| Proteins common between EPIC IM and the ECM of E17.5 | | Proteins common between EPIC SM and the ECM of E17.5 | | Proteins common between EPIC SM, EPIC IM and the ECM of E17.5 | |
|---|---|---|---|---|---|
| UniProt ID | Gene | UniProt ID | Gene | UniProt ID | Gene |
| P07724 | Alb | A2ASQ1-1 | Isoform 1 of Agrn | 008573 | Lgals9 |
| P10493 | Nid1 | E9Q414 | Apob | 088569 | Hnrnpa2b1 |
| Q3UQ28 | Pxdn | 008638-1 | Myh11 isoform 1 | P02468 | Lamc1 |
| Q8K0E8 | Fgb | 035604 | Npc1 | P02469 | Lamb1 |
| | | 035658 | C1qbp | P06745 | Gpi1 |
| | | 035887 | Calu | P07356 | Anxa2 |
| | | P02463 | Col4a1 | P08113 | Hsp90b1 |
| | | P08122 | Col4a2 | P10107 | Anxa1 |
| | | P14211 | Calr | P11087-1 | Col1a1 isoform 1 |
| | | P28653 | Bgn | P11152 | Lpl |
| | | P28654 | Dcn | P11276 | Fn1 |
| | | P29391 | Ftl1 | P11499 | Hsp90ab1 |
| | | P30681 | Hmgb2 | P13020-1 | Isoform 1 of Gsn |
| | | P31230 | Aimp1 | P17742 | Ppia |
| | | P39061-1 | Col18a 1 isoform 2 | P18760 | Cfl1 |
| | | P40124 | Cap1 | P20029 | Hspa5 |
| | | P51655 | Gpc4 | P21956-1 | Isoform 1 of Mfge8 |
| | | P63094 | Gnas | P27773 | Pdia3 |
| | | P63158 | Hmgb1 | P28301 | Lox |
| | | Q02788 | Col6a2 | P35441 | Thbs1 |
| | | Q04857 | Col6a1 | P37889 | Fbln2 |
| | | Q3V1T4 | P3h1 | P62962 | Pfn1 |
| | | Q5SS80-1 | Isoform 1 of Dhrs13 | P97792-1 | Isoform 1 of Cxadr |
| | | Q60847 | Col12a1 | Q01149 | Col1a2 |
| | | Q61207 | Psap | Q05793 | Hspg2 |
| | | Q62087 | Pon3 | Q61001 | Lama5 |
| | | Q62165 | Dag1 | Q61292 | Lamb2 |
| | | Q6GU68 | Islr | Q61703 | Itih2 |
| | | Q8CC88 | Vwa8 | Q62351 | Tfrc |
| | | Q8CIE6 | Cup | Q8CG19-1 | Long isoform of Ltbp1 |
| | | Q8K4Z3 | Naxe | Q8VDD5 | Myh9 |
| | | Q8VDL4 | Adpgk | Q99K41 | Emilin1 |
| | | Q91ZA3 | Pcca | Q9CYL5 | Glipr2 |
| | | Q99MN1 | Kars | | |
| | | Q9CYD3 | Crtap | | |
| | | Q9CZD3 | Gars | | |
| | | Q9D0L4-1 | Adck1 isoform 1 | | |
| | | Q9DCZ4 | Apoo | | |
| | | Q9JKR6 | Hyou1 | | |
| | | Q9QZF2 | Gpc1 | | |
| | | Q9WTR5 | Cdh13 | | |
| | | Q9Z2W0 | Dnpep | | |

The presence of fibronectin and laminin in E17.5 de-cellularised hearts was confirmed by Western Blot, performed as detailed in Example 3 (Fig. 4), where it is shown that fibronectin remains in detectable abundances.

Our analysis demonstrates that both fractions retain the key basement membrane (BM) proteins found in the heart, including laminins, collagen IV, agrin, perlecan, fibulin-1, nidogen-1, and SPARC, for example. Key ECM proteins are common to both decellularised embryonic hearts and EPIC-ECM, such as laminin (laminin α5, laminin β2 and β1, and laminin γ1), fibulin-2, agrin, and perlecan. Another protein found in common between EPIC-ECM and embryonic cardiac ECM is fibronectin. Specifically, in EPIC-ECM, fibronectin is mainly enriched in this fraction. The enrichment of ECM in fibronectin during cardiac development is associated with its essential role in cardiac vascular morphogenesis and repair in different organisms. In injured zebrafish hearts, the activated epicardium induces fibronectin expression at the injury site, to which cardiomyocytes respond by increasing the expression of integrin β3 (Itgb3), a protein that was also identified in the EPIC SM and IM fractions.

Annexin A2 is also present in EPIC SM, IM and embryonic hearts. The ability of annexin A2 to bind to plasminogen is known to be fulfilled when it forms a complex with S100a10, which is also identified in EPIC SM. The conversion of plasminogen by Anxa2/S100a10 to plasmin initiates a downstream proteolytic cascade that increases the expression of Mmp2 and Mmp9. Furthermore, in tumour cells, Anxa2 is commonly used to measure the invasive capacity of cancer cells. These results suggest that EPIC-derived ECM may play a bioactive role by modulating signalling through structural, proteolytic, and ligand/receptor signals.

Among the most abundant proteins in EPIC-IM we found the serine protease A1 (Htra1), which is required for the high temperature. Htra1 appears to be an important protein for ECM homeostasis and turnover, as it degrades several matrix components, including decorin, fibronectin, aggrecan, and type II and VI α1 collagens. Htra1 also degrades structural constituents of the basement membrane, such as nidogen-1, present in EPIC IM, and nidogen-2. In addition, Htra1 induces the expression of the protein A disintegrin and metalloproteinase with motifs of thrombospondin 5 (Adamts5) through the extracellular signal-regulated kinase (ERK), nuclear factor-κB (NF-κB), and c-Jun N-terminal kinase (JNK) signalling pathways. The latter molecule is a metalloproteinase that is also found in EPIC IM. The regulated activity of Htra1 is responsible for inducing proteolytic activity in ECM complexes.

### EXAMPLE 7

*The ECM obtained from EPIC shows* a *significantly different composition to Matrigel^{®}.*

The ECM derived from EPIC is also compared to Matrigel^{®}, as proof of concept in BM.

In this example, we demonstrate that EPIC-ECM contains proteins in common with Matrigel^{®}. The workflow of the *in silico* analysis used was as in examples 4 and 7. A total of 309 proteins are identified in Matrigel^{®}. Thirty-three proteins are common to Matrigel^{®}, EPIC IM and EPIC SM, while 69 proteins are shared exclusively between EPIC IM and Matrigel^{®} and another 69 proteins are shared only between Matrigel^{®} and EPIC SM. 89 proteins were identified in Matrigel^{®}. Of these, 21 proteins are commonly found in EPIC SM and EPIC IM, as shown in Table 5.

As for extracellular proteins shared exclusively between EPIC IM and Matrigel^{®}, 7 proteins are identified. As for proteins exclusive to EPIC SM and Matrigel^{®}, 20 proteins are identified (Table 5; Fig. 10). In total, EPIC-ECM shares 48 extracellular proteins with Matrigel^{®}.

**Table 5. Extracellular proteins common to Matrigel^{®} and EPIC IM, EPIC SM proteins common to all conditions.**

| Proteins common between EPIC IM and Matrigel^{®} | | Proteins common between EPIC SM and Matrigel^{®} | | Proteins common between EPIC SM, EPIC IM and Matrigel^{®} | |
|---|---|---|---|---|---|
| UniProt ID | Gene | UniProt ID | Gene | UniProt ID | Gene |
| A6X935-1 | Isoform 1 of Itih4 | A2ASQ1-1 | Isoform 1 of Agrn | 088569 | Hnrnpa2b1 |
| P01027-1 | Long isoform of C3 | 035887 | Calu | P02468 | Lamc1 |
| P07724 | Alb | P02463 | Col4a1 | P02469 | Lamb1 |
| P10493 | Nid1 | P07214 | Sparc | P07356 | Anxa2 |
| P19137 | Lama1 | P08122 | Col4a2 | P08113 | Hsp90b1 |
| Q3UQ28 | Pxdn | P14211 | Calr | P11276 | Fn1 |
| Q8K0E8 | Fgb | P18242 | Ctsd | P11499 | Hsp90ab1 |
| | | P28653 | Bgn | P13020-1 | Isoform 1 of |
| | | | | | Gsn |
| | | P29391 | Ftl1 | P16110 | Lgals3 |
| | | P39061-1 | Col18a1 isoform 2 | P17742 | Ppia |
| | | P55302 | Lrpap1 | P20029 | Hspa5 |
| | | P63158 | Hmgb1 | P27773 | Pdia3 |
| | | Q3V1T4 | P3h1 | P62960 | Ybx1 |
| | | Q60847 | Col12a1 | P62962 | Pfn1 |
| | | Q61147 | Cp | Q05793 | Hspg2 |
| | | Q61207 | Psap | Q08879 | Fbln1 |
| | | Q99JR5 | Tinagl1 | Q61001 | Lama5 |
| | | Q9CYD3 | Crtap | Q61292 | Lamb2 |
| | | Q9JKR6 | Hyou1 | Q8VDD5 | Myh9 |
| | | Q9Z204 | Hnrnpc | Q9CYL5 | Glipr2 |
| | | | | Q9WU78 | Pdcd6ip |

The presence of fibronectin and laminin in Matrigel^{®} was confirmed by Western Blot, performed as detailed in Example 3 (Fig. 4), which shows the presence of fibronectin in relatively low abundance, while laminin is very abundant in this sample.

### EXAMPLE 8

### The composition of EPIC-IM stimulates endothelial cell proliferation

Analysis of the composition of ECM derived from EPIC and Matrigel^{®} provides strong evidence of the potential of this ECM to mimic the cellular support provided by Matrigel^{®}. Taking this characteristic into account, an *in vitro* assay was performed to allow a functional comparison between ECM fractions derived from Matrigel^{®} and EPIC in human umbilical vein endothelial cells (HUVECs).

This example illustrates the proliferation of endothelial cells across plastic surfaces coated with EPIC IM and their angiogenic potential. A primary culture of HUVECs was used to determine the ability of the EPIC IM components described in this document to induce proliferation in endothelial cells.

The ability of HUVECs to form tubular structures in Matrigel^{®} has been widely demonstrated. To do so, this primary human cell culture requires a supplemental surface coating to ensure spontaneous proliferation. For this reason, 1% gelatine was used to allow direct comparison between the experimental and control groups.

In the design of our study, two time points (3 and 5 days) were considered to capture the proliferative window of HUVECs after seeding. One of the characteristics that changes visibly is the organisation of HUVECs at the time of analysis (Fig. 11). For surface coating, the matrix solutions were placed on the plastic surface to cover it completely. The matrices containing the surface were incubated for two hours at 37°C, the excess solution was aspirated, and the surfaces were allowed to dry. HUVECs were deposited in wells coated with EPIC IM or EPIC SM for three and five days in endothelial growth medium (Lonza) at 37°C and 5% CO₂. For comparison, HUVECs were also deposited on Matrigel^{®}, Matrigel^{®} with EPIC IM, and EPIC IM with EPIC SM. The fact that differences with Matrigel^{®} appear later (Day 5 vs Day 3) demonstrates the angiogenic potency of EPIC-IM, reinforcing its characteristics as a proangiogenic and proliferative matrix.

Proliferation rates were assessed at the end of the assay incubation periods by labelling S-phase activation through EdU incorporation into DNA. Next, paraformaldehyde-fixed cells underwent a *Click-It* chemical reaction in which the EdU alkyne manipulator binds to azide-containing molecules and allows the detection of proliferative cells by fluorescent microscopy. Statistical analysis was performed using a two-way ANOVA test with Tukey's corrections. Results were considered significant when p < 0.05.

In the analysis performed after three days of culture, a trend toward reduced proliferation was observed in all treatments compared to gelatine- (Fig. 11). However, only HUVECs cultured with EPIC-SM or Matrigel^{®} showed a statistically significant reduction compared to the control condition.

After five days of culture, the surfaces coated with gelatine and EPIC IM showed the highest incorporation of EdU compared to all the conditions tested, being significantly higher than the wells coated with EPIC SM (Fig. 12). Although statistical significance was not achieved compared to wells coated with Matrigel^{®} or EPIC IM with EPIC SM, proliferation rates were lower than in negative controls; only EPIC IM showed proliferation similar to that of gelatine. These results reinforce the possible angiogenic role introduced by EPIC IM that was not observed in EPIC SM. Culture conditions containing Matrigel^{®} behave very similarly to each other. However, an improvement in the proliferation rate of HUVECs is observed when EPIC IM is combined with Matrigel^{®}, as HUVECs proliferate more on day 5 than on Matrigel^{®}-coated surfaces (Fig. 12).

On plastic surfaces coated with Matrigel^{®} and EPIC IM, the arrangement of HUVECs was more similar to that in wells coated with Matrigel^{®} than to that in wells coated with EPIC IM. On surfaces coated with EPIC SM, the seeded HUVECs were mostly scattered and isolated, although some cells already showed a circular-type organisation. As for the ECM surfaces derived from EPIC (surfaces coated with EPIC SM and EPIC IM), a similar cellular organisation to that of HUVECs was observed on surfaces coated with EPIC SM (Fig. 13). One possible explanation is that EPIC-SM dilutes the protein content of EPIC-IM, which affects the increase in proliferative capacity.

## Claims

1. A murine extracellular matrix (ECM) obtained from the EPIC cell line, said cell line being deposited at the Leibniz Institute DSMZ and with a deposit number DSM ACC3373.

2. The murine extracellular matrix according to the previous claim comprising a soluble or insoluble fraction.

3. A method for obtaining the murine extracellular matrix defined in any of the claims comprising mechanical and/or chemical disruption of the EPIC cell line with deposit number DSM ACC3373.

4. The method according to the previous claim wherein the chemical disruption is performed with NH₄OH.

5. The method according to the previous claim comprising the following steps:
1) de-cellularisation of the EPIC cell line with deposit number DSM ACC3373 adhered to a culture plate by means of an NH₄OH solution for at least 5 minutes
2) collecting and discarding the NH₄OH solution from the previous step such that the murine extracellular matrix ECM is located on said culture plate.

6. The method according to claim 4 comprising the following steps:
1) de-cellularisation of the EPIC cell line with deposit number DSM ACC3373 adhered to a culture plate by means of an NH₄OH solution for at least 5 minutes
2) collecting and discarding the NH₄OH solution from the previous step
3) dragging the gelatinous phase from the plate to obtain the soluble fraction
4) washing the bottom of the culture plate to obtain the insoluble fraction
where steps 1-4 must be performed in the order indicated
such that the murine extracellular matrix fractions are collected separately outside the culture plate.

7. The method according to one of the preceding claims 5 or 6, wherein step 1) is performed after culturing the EPIC cell line with deposit number DSM ACC3373 for a period of between 4 days and 15 days.

8. The method according to any of claims 5 to 7, wherein step 1) is performed after a period of time between 1 day and 7 days once the culture of the EPIC cell line with deposit number DSM ACC3373 reaches a confluence of between 80% and 100%.

9. Use of the murine extracellular matrix defined in any of claims 1 or 2, or obtained according to the method of claims 3 to 8, for the culture of a cell line, comprising contacting said cell line with the ECM obtained from the EPIC cell line with deposit number DSM ACC3373, alone or accompanied by gelatine suitable for cell cultures.

10. Use according to the previous claim, wherein the culture comprises an increase in proliferation and/or angiogenesis of the cell line compared to normal levels of proliferation and/or angiogenesis of said cell line.

11. Use according to one of claims 9 or 10, wherein the culture is *in vitro.*

12. Use according to one of claims 9 to 11, wherein the insoluble fraction of the murine extracellular matrix is employed.

13. Use according to one of claims 9 to 11, wherein the soluble fraction of the murine extracellular matrix is employed.

14. Use according to one of claims 9 to 13, wherein a mixture of the insoluble fraction and the soluble fraction of the murine extracellular matrix is used.

15. Use according to any of the preceding claims 9 to 14, wherein the cell line is of human or murine origin.

16. Use according to the preceding claim, wherein the cell line is selected from HUVEC, AC16 and HL-1.
